(19) Europäisches Patentamt / European Patent Office / Office européen des brevets

(11) **EP 4 404 206 A1**

(12) **EUROPEAN PATENT APPLICATION**
published in accordance with Art. 153(4) EPC

(43) Date of publication:
**24.07.2024 Bulletin 2024/30**

(21) Application number: **22870218.9**

(22) Date of filing: **08.09.2022**

(51) International Patent Classification (IPC):
*G16H 30/40* (2018.01)   *G16H 30/20* (2018.01)
*G16H 50/50* (2018.01)   *G16H 50/20* (2018.01)
*A61B 8/08* (2006.01)   *A61B 6/00* (2024.01)
*A61B 5/055* (2006.01)

(52) Cooperative Patent Classification (CPC):
**A61B 5/055; A61B 6/00; A61B 8/08; G16H 30/20;
G16H 30/40; G16H 50/20; G16H 50/50**

(86) International application number:
**PCT/KR2022/013534**

(87) International publication number:
**WO 2023/043133 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.09.2021 KR 20210125252**

(71) Applicant: **Ontact Health Co., Ltd.**
**Seoul 03764 (KR)**

(72) Inventors:
• **SHIM, Hack Joon**
 **Seoul 06214 (KR)**
• **HONG, Young Taek**
 **Seoul 04094 (KR)**
• **JEONG, Da Wun**
 **Gunpo-si Gyeonggi-do 15861 (KR)**
• **CHOI, Annes**
 **Seoul 04193 (KR)**

(74) Representative: **Peterreins Schley**
**Patent- und Rechtsanwälte PartG mbB**
**Hermann-Sack-Straße 3**
**80331 München (DE)**

(54) **METHOD AND DEVICE FOR DETERMINING ABNORMALITIES IN ORGANS OR MUSCLES IN BODY**

(57)    In order to determine presence or absence of abnormality in an internal organ or muscle, a method of determining presence or absence of abnormality in an internal organ or muscle may comprise obtaining at least one image of the internal organ or muscle, determining at least one feature matrix for the at least one image, determining a feature value for measuring a tissue phenotype from the at least one feature matrix, and determining presence or absence of the abnormality in the internal organ or muscle using a trained artificial intelligence model. The feature value may comprise items selected as information indicating the tissue phenotype of the internal organ or muscle, and the items may be selected using a machine learning model among candidate feature values determinable from the at least one feature matrix.

FIG. 5

EP 4 404 206 A1

**Description**

**Technical Field**

The present disclosure relates to determination of the status of an internal organ or muscle, and more particularly, to a method and device for determining presence or absence of abnormality in an internal organ or muscle.

**Background Art**

Disease refers to a condition that causes disorders in the human mind and body, impeding normal functions. Depending on the disease, a person may suffer and may even be unable to preserve their life. Accordingly, various social systems and technologies for diagnosing, treating, and even preventing diseases have developed along with human history. In the diagnosis and treatment of diseases, various tools and methods have been developed in accordance with the remarkable advancement of technology, but the reality is that they are still ultimately dependent on the judgment of doctors.

Meanwhile, recently, artificial intelligence (AI) technology has developed significantly and is attracting attention in various fields. In particular, due to the environment such as vast amounts of accumulated medical data and image-oriented diagnostic data, various attempts and research are underway to apply artificial intelligence algorithms to the medical field. Specifically, various studies are being conducted to use artificial intelligence algorithms to solve tasks that have traditionally been dependent on clinical judgment, such as diagnosing and predicting diseases.

**Disclosure**

**Technical Problem**

An object of the present disclosure is to provide a method and device for effectively determining presence or absence of abnormality in an internal organ or muscle using an artificial intelligence (AI) algorithm.

An object of the present disclosure is to provide a method and device for effectively diagnosing presence or absence of abnormality in an internal organ or muscle using an artificial intelligence algorithm.

An object of the present disclosure is to provide a method and device for effectively determining presence or absence of abnormality in an internal organ or muscle based on images.

An object of the present disclosure is to provide a method and device for effectively diagnosing presence or absence of abnormality in an internal organ or muscle based on images.

An object of the present disclosure is to provide a method and device for determining presence or absence of abnormality in an internal organ or muscle based on information on a tissue phenotype obtained from medical images of the heart.

An object of the present disclosure is to provide a method and device for diagnosing presence or absence of abnormality in an internal organ or muscle based on information on a tissue phenotype obtained from medical images of the heart.

An object of the present disclosure is to provide a method and device for determining or diagnosing presence or absence of abnormality in an internal organ or muscle using some feature values selected by a machine learning model among feature values that may be obtained from medical images of the heart.

The technical problems solved by the present disclosure are not limited to the above technical problems and other technical problems which are not described herein will be clearly understood by a person (hereinafter referred to as an ordinary technician) having ordinary skill in the technical field, to which the present disclosure belongs, from the following description.

**Technical Solution**

A method of determining presence or absence of abnormality in an internal organ or muscle according to an embodiment of the present disclosure may comprise obtaining at least one image of the internal organ or muscle, determining at least one feature matrix for the at least one image, determining a feature value for measuring a tissue phenotype from the at least one feature matrix, and determining presence or absence of the abnormality in the internal organ or muscle using a trained artificial intelligence model. The feature value may comprise items selected as information indicating the tissue phenotype of the internal organ or muscle, and the items may be selected using a machine learning model among candidate feature values determinable from the at least one feature matrix.

According to an embodiment of the present invention, the at least one image may comprise at least one of ultrasound, computed tomography (CT), positron emission tomography (PET), or magnetic resonance imaging (MRI).

According to an embodiment of the present invention, the at least one image may comprise an image captured in at least one view of apical 2-chamber (A2CH), apical 4-chamber (A4CH), parasternal long-axis (PLAX), parasternal short-axis (PSAX)-mid, PLAX increased depth, parasternal long axis left ventricle (PLAX LV), parasternal long axis left ventricle aorta/left atrial (PLAX LV-AO/LA), parasternal long axis zoomed left ventricle (PLAX zoomed LV), parasternal long axis zoomed aortic valve (PLAX zoomed AV), parasternal long axis zoomed mitral valve (PLAX zoomed MV), parasternal long axis right ventricle (PLAX RV) out flow, parasternal long axis right ventricle (PLAX RV) inflow, parasternal short axis (level great vessels) focus on pulmonic valve (PSAX focus on PV), parasternal short axis (level great vessels) focus on aortic valve (PSAX focus on AV), parasternal short axis (level great vessels) zoomed aortic valve(PSAX zoomed AV), parasternal short axis (level great vessels) focus on tricuspid valve (PSAX (level great vessels) focus on TV), parasternal short axis focus on pulmonic valve and pulmonary artery (PSAX focus on PV and PA), parasternal short axis-level of mitral valve (PSAX-level of MV), parasternal short axis(PSAX)-level of papillary muscles, parasternal short axis (PSAX)-level of apex, A5C (apical five-chamber), A4C (apical four-chamber), apical four-chamber zoomed left ventricle (A4C zoomed LV), apical four-chamber right ventricle(A4C RV-focused)-focused, apical four-chamber (A4C) posterior angulation), apical three-chamber (A3C), apical three-chamber zoomed left ventricle (A3C zoomed LV), apical two-chamber (A2C), apical two-chamber zoomed left ventricle (A2C zoomed LV), apical long axis, apical long axis zoomed left ventricle (LV), apical four-chamber left atrial pulmonary vein focus (A4C LA Pulvn), subcostal four-chamber (SC 4C), subcostal long axis inferior vena cava (SC long axis IVC), subcostal window hepatic vein (SC window Hvn), or suprasternal notch (SSN) aortic arch.

According to an embodiment of the present invention, the at least one feature matrix may be determined based on pixel values included in a region of interest extracted from the at least one image.

According to an embodiment of the present invention, the region of interest may comprise at least one selected from a group consisting of an outer wall, medial wall, parietal peritoneum, fascia, intervisceral space, intracavitary, and intraperitoneal viscera, skeletal muscle, myocardium, and smooth muscle.

According to an embodiment of the present invention, the at least one feature matrix may comprise at least one of gray-level co-occurrence matrix (GLCM), gray-level run length matrix (GLRLM), gray-level size zone matrix (GLSZM), neighborhood gray-tone difference matrix (NGTDM) or gray level dependence matrix (GLDM).

According to an embodiment of the present invention, the abnormality in the internal organ or muscle may comprise heart disease.

A device for determining presence or absence of abnormality in an internal organ or muscle according to an embodiment of the present disclosure may comprise a transceiver, a storage unit configured to store an artificial intelligence model, and at least one processor connected to the transceiver and the storage unit. The at least one processor may obtain at least one image of the internal organ or muscle, determine at least one feature matrix for the at least one image, determine a feature value for measuring a tissue phenotype from the feature matrix, and determine presence or absence of the abnormality in the internal organ or muscle using a trained artificial intelligence model. The feature values may comprise items selected as information indicating the tissue phenotype in the internal organ or muscle, and the items may be selected using a machine learning model among candidate feature values determinable from the at least one feature matrix.

A program stored on a medium according to an embodiment of the present invention may execute the above-described method when operated by a processor.

The features briefly summarized above with respect to the present disclosure are merely exemplary aspects of the detailed description of the present disclosure described below, and do not limit the scope of the present disclosure.

**Advantageous Effects**

According to the present invention, presence or absence of abnormality in an internal organ or muscle can be effectively diagnosed using a trained artificial intelligence model.

It will be appreciated by persons skilled in the art that that the effects that can be achieved through the present disclosure are not limited to what has been particularly described hereinabove and other advantages of the present disclosure will be more clearly understood from the detailed description.

**Description of Drawings**

The above and other objects, features and other advantages of the present invention will be more clearly understood from the following detailed description taken in conjunction with the accompanying drawings, in which:

FIG. 1 shows a system according to an embodiment of the present invention;
FIG. 2 shows the structure of a device for predicting the possibility of disease occurrence according to an embodiment of the present invention;

FIG. 3 shows an example of a random forest model applicable to the present invention;

FIGS. 4A to 4E show examples of feature matrices determined from an image according to an embodiment of the present invention;

FIG. 5 shows an example of a procedure for determining presence or absence of abnormality in an internal organ or muscle according to an embodiment of the present invention;

FIG. 6 shows an example of a random forest model for selecting feature items according to an embodiment of the present invention;

FIG. 7 shows an example of a procedure for selecting feature items for diagnosis of heart disease according to an embodiment of the present invention;

FIG. 8 shows an example of a procedure for diagnosing heart disease using an artificial intelligence model according to an embodiment of the present invention;

FIGS. 9A to 9D show validation results for an artificial intelligence model according to a 10-fold cross validation method when using an image of an end systole phase according to an embodiment of the present invention;

FIGS. 10A to 10D show verification results for an artificial intelligence model according to an LOO cross validation method when using an image of the end systole phase according to an embodiment of the present invention;

FIGS. 11A to 11D show verification results for an artificial intelligence model according to a 10-fold cross validation method when using an image of an end diastole phase according to an embodiment of the present invention;

FIGS. 12A to 12D show verification results for an artificial intelligence model according to an LOO cross validation method when using an image of an end diastole phase according to an embodiment of the present invention;

FIGS. 13A to 13D show verification results for an artificial intelligence model according to a 10-fold cross validation method when using a mid-phase image according to an embodiment of the present invention;

FIGS. 14A to 14D show verification results for an artificial intelligence model according to an LOO cross validation method when using an image of a mid-phase according to an embodiment of the present invention;

FIG. 15 shows verification results for an artificial intelligence model according to an 10-fold cross validation method when using an image of all phases according to an embodiment of the present invention; and

FIG. 16 shows verification results for an artificial intelligence model according to an LOO cross validation method when using an image of all phases according to an embodiment of the present invention.

## Mode for Invention

Hereinafter, with reference to the accompanying drawings, embodiments of the present disclosure will be described in detail so that those skilled in the art can easily practice them. However, the present disclosure may be implemented in many different forms and is not limited to the embodiments described herein.

In describing embodiments of the present disclosure, if it is determined that detailed descriptions of known configurations or functions may obscure the subject matter of the present disclosure, detailed descriptions thereof will be omitted. In addition, in the drawings, parts that are not related to the description of the present disclosure are omitted, and similar parts are given similar reference numerals.

The present invention is to diagnose abnormality in an internal organ or muscle using an artificial intelligence algorithm. Specifically, it relates to a technology for diagnosing abnormality in an internal organ or muscle based on images.

In the following description, the internal organ or muscle refers to at least one selected from a group consisting of an outer wall, medial wall, parietal peritoneum, fascia, intervisceral space, intracavitary, intraperitoneal viscera, skeletal muscle, myocardium, and smooth muscle.

Additionally, in the following description, abnormality in an internal organ or muscle refers to heart disease, and more specifically, to myocardial disease. For example, abnormality in the internal organ or muscle may include at least one of a group consisting of cardiac hypertrophy, hypertrophic cardiomyopathy (HCM), dilated cardiomyopathy (DCM), heart failure with preserved ejection fraction (HFpEF), diastolic dysfunction of the heart, cardiomyopathy including primary or secondary restrictive cardiomyopathy, myocardial infarction, cardiac fibrosis, sarcomere mutations, that is, myosin heavy chain $\beta$ (MHC-$\beta$), cardiac muscle troponin T (cTnT), tropomyosin alpha-1 chain (TPM1), and myosin-binding protein C cardiotype (MYBPC3), cardiac troponin I (cTnI), myosin essential light chain (ELC), titin (TTN), myosin regulatory light chain 2 ventricular/cardiac muscle isoform (MLC-2), cardiac muscle alpha-actin, myocardial abnormality due to mutations in muscle LIM protein (MLP) or protein kinase AMP-activating non-catalytic subunit gamma 2 (PRKAG2), Fabry disease, Danon disease, mitochondrial cardiomyopathy, Noonan syndrome, hypertension, valvular heart disease (e.g., aortic stenosis and mitral valve) pulmonary failure), metabolic syndrome (such as diabetes and obesity), end-stage renal disease, scleroderma, sleep apnea, amyloidosis, Friedreich's ataxia, myocardial abnormalities due to Danon disease, or Pompe disease, angina pectoris, left ventricular outflow tract dysfunction, hypertensive heart disease, congenital heart disease, cardiac ischemia and/or coronary heart disease, diabetic heart disease, congestive heart failure, right-sided heart failure, infiltrative cardiomyopathy, cardiorespiratory syndrome, myocardial abnormalities due to cardiac aging or diastolic dysfunction due to aging or due to conditions related thereto, or myocardial abnormality due to left ventricular hypertrophy and/or concentric left ventricular remodeling, or due to conditions related thereto.

In various embodiments of the present invention, which will be described later, a subj ect for which presence or absence of abnormality in an internal organ or muscle will be determined is a mammal. In some specific examples, the subject may be a mouse, rat, dog, cat, pig, sheep, horse, cow, or human. In some embodiments, the subject may be a human.

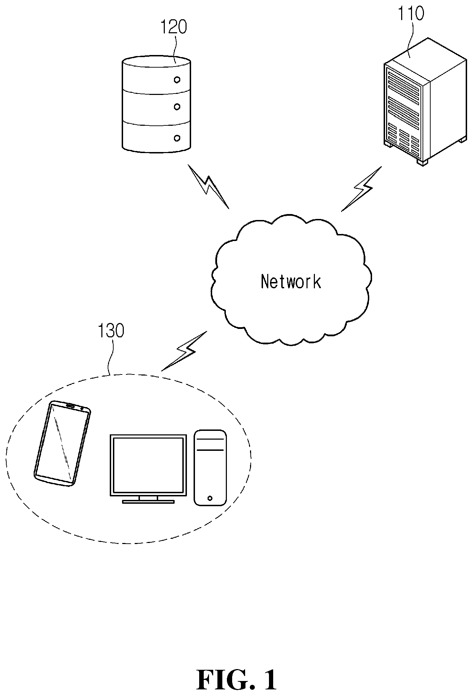

FIG. 1 shows a system according to an embodiment of the present invention.

Referring to FIG. 1, the system includes a service server 110, a data server 120, and at least one client device 130.

The service server 110 provides services based on artificial intelligence models. That is, the service server 110 performs learning and prediction operations using artificial intelligence models. The service server 110 may communicate with the data server 120 or at least one client device 130 through a network. For example, the service server 110 may receive learning data for training an artificial intelligence model from the data server 120 and perform training. The service server 110 may receive data required for learning and prediction operations from at least one client device 130. Additionally, the service server 110 may transmit information on the prediction result to at least one client device 130.

The data server 120 provides learning data for training the artificial intelligence model stored in the service server 110. According to various embodiments, the data server 120 may provide public data that anyone can access or provide data that requires permission. If necessary, the learning data may be preprocessed by the data server 120 or the service server 110. According to another embodiment, the data server 120 may be omitted. In this case, the service server 110 may use an externally trained artificial intelligence model, or learning data may be provided to the service server 110 offline.

The at least one client device 130 transmits and receives data related to the artificial intelligence model operated by the service server 110 to and from the service server 110. The at least one client device 130 is used by a user, and transmits information input by the user to the service server 110, and stores the information received from the service server 110 or provides (shows) it to the user. In some cases, a prediction operation may be performed based on data transmitted from one client, and information related to the result of prediction may be provided to another client. The at least one client device 130 may be various types of computing devices, such as desktop computers, laptop computers, smartphones, tablets, and wearable devices.

Although not shown in FIG. 1, the system may further include a management device for managing the service server 110. The management device is a device used by an entity that manages the service, and monitors the status of the service server 110 or controls settings of the service server 110. The management device may be connected to the service server 110 through a network or directly through a cable connection. According to the control of the management device, the service server 110 may set parameters for operation.

As described with reference to FIG. 1, the service server 110, the data server 120, the at least one client device 130, the management device, etc. may be connected and interact through the network. Here, the network may include at least one of a wired network or a wireless network, and may be composed of any one or a combination of two or more of a cellular network, a local area network, and a wide area network. For example, the network may be implemented based on at least one of local area network (LAN), wireless LAN (WLAN), Bluetooth, long term evolution (LTE), LTE-advanced (LTE-A), or 5th generation (5G).

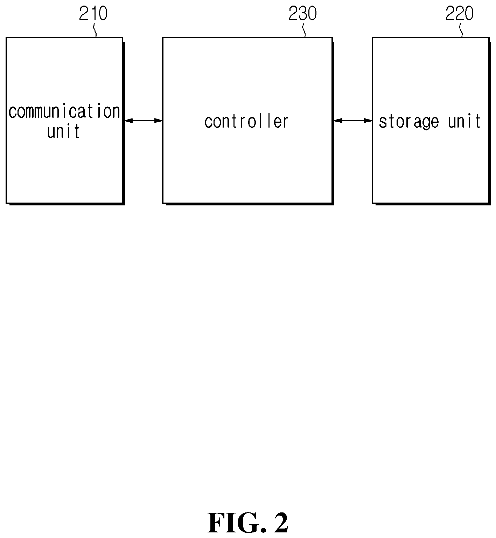

FIG. 2 shows the structure of a device for predicting the possibility of disease occurrence according to an embodiment of the present invention. The structure illustrated in FIG. 2 may be understood as the structure of the service server 110, data server 120, and at least one client device 130 of FIG. 1.

Referring to FIG. 2, the device includes a communication unit 210, a storage unit 220 and a controller 230.

The communication unit 210 performs functions to connect to the network and communicate with other devices. The communication unit 210 may support at least one of wired communication or wireless communication. For communication, the communication unit 210 may include at least one of a radio frequency (RF) processing circuit or a digital data processing circuit. In some cases, the communication unit 210 may be understood as a component including a terminal for connecting a cable. Since the communication unit 210 is a component for transmitting and receiving data and signals, it may be referred to as a 'transceiver'.

The storage unit 220 stores data, programs, microcode, instruction sets, applications, etc. necessary for the operation of the device. The storage unit 220 may be implemented as a transitory or non-transitory storage medium. Additionally, the storage unit 220 may be fixed to the device or may be implemented in a detachable form. For example, the storage unit 220 may be implemented as at least one of NAND flash memories such as compact flash (CF) cards, secure digital (SD) cards, memory sticks, solid-state drives (SSDs) or micro SD cards or magnetic computer storage devices such as hard disk drives (HDDs).

The controller 230 controls the overall operation of the device. To this end, the controller 230 may include at least one processor, at least one microprocessor, etc. The controller 230 may execute a program stored in the storage unit 220 and access the network through the communication unit 210. In particular, the controller 230 may perform algorithms according to various embodiments described later and control the device to operate according to embodiments described later.

An artificial intelligence model that may be used to solve various problems such as detection, classification, and regression may include random forest. Random forest is an ensemble artificial intelligence model composed of a plurality of decision trees, and was proposed to solve problems such as overfitting and error propagation that may occur when using a single decision tree.

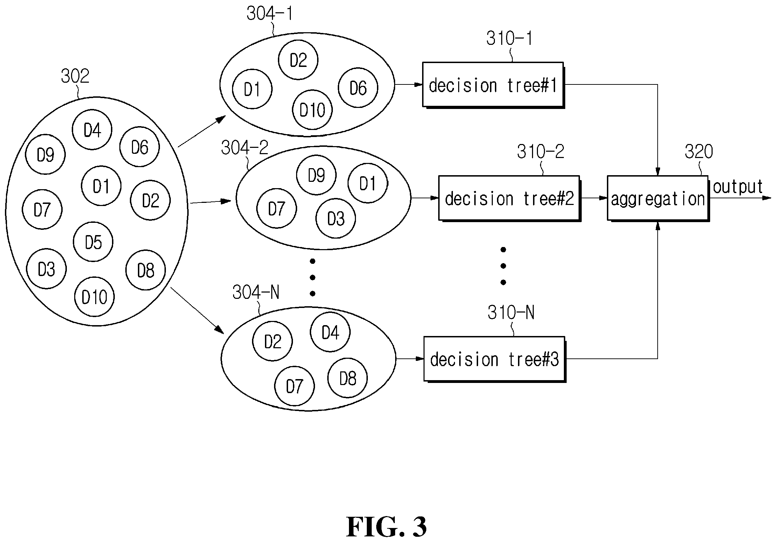

FIG. 3 shows an example of a random forest model applicable to the present invention. Referring to FIG. 3, the random forest model includes a plurality of decision trees 310-1 to 310-N and an aggregation network 320. Each of the decision trees 310-1 to 310-N performs a classification operation using one of subsets 304-1 to 304-N of the entire data set 302. Then, the classification results of each of the decision trees 310-1 to 310-N are aggregated by the aggregation network 320, thereby generating one output. For example, the aggregation network 320 may provide, as output, the result with the largest number of votes among the classification results from the decision trees 310-1 to 310-N.

Here, operation of dividing the data set 302 into subsets 304-1 to 304-N is referred to as bagging. At this time, since the subsets 304-1 to 304-N do not have to be mutually exclusive, a plurality of subsets (e.g., subset #1 304-1, subset #2 304-2) include a common data element (e.g., D1). In other words, since the bagging operation allows duplicate selection of data, even if more than M subsets are generated so that each subset includes N/M data elements in a data set containing N data elements, they may all be unique subsets.

Through training based on bagging, the decision trees 310-1 to 310-N are randomized. Through randomization, the decision trees 310-1 to 310-N forming the random forest model may have different characteristics due to randomness. Differences in characteristics cause decorrelation between predictions made by the decision trees 310-1 to 310-N, and as a result, contribute to improvement of the generalization performance of the random forest model. Additionally, the randomization characteristics provide robustness against noise of the random forest model.

The decision trees 310-1 to 310-N included in the random forest model are composed of a plurality of nodes including questions for classification. Generally, each node contains a question for binary classification, and the content of the question may be generated through training. Therefore, training a decision tree may be understood as a process of generating a list of questions. The learning algorithm for a decision tree builds a tree by selecting an item that most closely classifies a target result from all possible tests and repeating this. This process may be repeated until a leaf node becomes a pure node. At this time, to prevent the depth of the tree from becoming infinite, pruning may be performed.

Once the decision tree is completed, the importance of feature items corresponding to a node may be evaluated. Importance may be expressed by the Gini index, which indicates impurity. The lower the Gini index, the higher the importance. The Gini index indicates how concentrated the samples classified at a given node are in one class. For example, the Gini index of a pure node is 0.

The artificial intelligence algorithm according to various embodiments of the present invention uses medical images of the heart to diagnose abnormality in an internal organ or muscle. Specifically, the present invention may determine feature values from medical images and then use the feature values to diagnose presence or absence of abnormality in an internal organ or muscle. For example, the medical images may include images obtained from a view in which the myocardium of the left ventricle may be observed (e.g., at least one of apical 2-chamber (A2CH), apical 4-chamber (A4CH), parasternal long-axis (PLAX), parasternal short-axis (PSAX)-mid, PLAX increased depth, parasternal long axis left ventricle (PLAX LV), parasternal long axis left ventricle aorta/left atrial (PLAX LV-AO/LA), parasternal long axis zoomed left ventricle (PLAX zoomed LV), parasternal long axis zoomed aortic valve (PLAX zoomed AV), parasternal long axis zoomed mitral valve (PLAX zoomed MV), parasternal long axis right ventricle (PLAX RV) out flow, parasternal long axis right ventricle (PLAX RV) inflow, parasternal short axis (level great vessels) focus on pulmonic valve (PSAX focus on PV), parasternal short axis (level great vessels) focus on aortic valve (PSAX focus on AV), parasternal short axis (level great vessels) zoomed aortic valve(PSAX zoomed AV), parasternal short axis (level great vessels) focus on tricuspid valve (PSAX (level great vessels) focus on TV), parasternal short axis focus on pulmonic valve and pulmonary artery (PSAX focus on PV and PA), parasternal short axis-level of mitral valve (PSAX-level of MV), parasternal short axis(PSAX)-level of papillary muscles, parasternal short axis (PSAX)-level of apex, A5C (apical five-chamber), A4C (apical four-chamber), apical four-chamber zoomed left ventricle (A4C zoomed LV), apical four-chamber right ventricle(A4C RV-focused)-focused, apical four-chamber (A4C) posterior angulation), apical three-chamber (A3C), apical three-chamber zoomed left ventricle (A3C zoomed LV), apical two-chamber (A2C), apical two-chamber zoomed left ventricle (A2C zoomed LV), apical long axis, apical long axis zoomed left ventricle (LV), apical four-chamber left atrial pulmonary vein focus (A4C LA Pulvn), subcostal four-chamber (SC 4C), subcostal long axis inferior vena cava (SC long axis IVC), subcostal window hepatic vein (SC window Hvn), or suprasternal notch (SSN) aortic arch).

The feature values determined to diagnose presence or absence of abnormality in an internal organ or muscle are the values of items selected in advance to evaluate the texture of the tissue. For example, the feature values may be selected using the random forest model described above. The feature values that may provide texture information enough to diagnose heart disease may be selected from various candidate feature values. The candidate feature values may include feature values related to a shape that may be determined from a 3D image and a 2D image. Examples of feature values related to the shape that may be determined from the 3D image are shown in [Table 1] below, and feature values related to the shape that may be determined from the 2D image are shown in [Table 2] below.

[Table 1]

| | | Name | Description |
|---|---|---|---|
| | 1 | Mesh Volume | Volume calculated from a triangular mesh of a region of interest (ROI) |
| | 2 | voxel Volume | Approximated and measured by multiplying the number of voxels in the ROI by the volume of a single voxel |
| | 3 | Surface Area | Surface area calculated using the triangular mesh |
| | 4 | Surface Area to Volume ratio | The lower the value, the closer the shape is to sphericity |
| | 5 | Sphericity | Measurement of circularity of tumor shape for sphericity |
| | 6 | Compactness 1 | Measurement of how close the tumor shape is to sphericity compared to a perfect sphere (similar to sphericity) |
| | 7 | Compactness 2 | Similar to Compactness1, and dimensionless measurement independent of scale and direction |
| | 8 | Spherical Disproportion | Ratio of the surface area of the tumor to the surface area of sphericity with the same volume as the tumor site (inverse of Sphericity) |
| | 9 | Maximum 3D diameter | Maximum pairwise Euclidean distance between mesh vertices of the tumor surface |
| | 10 | Maximum 2D diameter (Slice) | Maximum pairwise Euclidean distance in the axial plane (row-column) |
| | 11 | Maximum 2D diameter (Column) | Maximum pairwise Euclidean distance in the coronal plane (row-slice) |
| | 12 | Maximum 2D diameter (Row) | Maximum pairwise Euclidean distance in the sagittal plane (column-slice) |
| | 13 | Major Axis Length | Calculate the largest axis length of the ellipsoid surrounding the ROI using the largest principal component |
| | 14 | Minor Axis Length | Calculate the second largest axis length of the ellipsoid surrounding the ROI using the largest principal component |
| | 15 | Least Axis Length | Calculate the smallest axis length of the ellipsoid surrounding the ROI using the largest principal component |
| | 16 | Elongation | Relationship between the two largest principal components in the ROI shape |
| | 17 | Flatness | Show the relationship between the largest and smallest principal components in the ROI shape |

[Table 2]

| | | Name | Description |
|---|---|---|---|
| | 1 | Mesh Surface | Surface area calculated from the triangular mesh of ROI |
| | 2 | Pixel Surface | Value approximated by multiplying the number of pixels in ROI by the surface area of single pixel |
| | 3 | Perimeter | Perimeter value in mesh |
| | 4 | Perimeter to Surface ratio | Value indicating a degree close to a circle (the close it is to a circle, the lower the value) |
| | 5 | Sphericity | Measurement of circularity of tumor shape relative to shape |
| | 6 | Spherical Disproportion | Ratio of the surface area of the tumor to the surface area of a sphere with the same volume as the tumor site (inverse of sphericity) |

(continued)

|  | Name | Description |
|---|---|---|
| 7 | Maximum 2D diameter | Maximum diameter |
| 8 | Major Axis Length | Major axis length |
| 9 | Minor Axis Length | Minor axis length |
| 10 | Elongation | Relationship between two largest principal components in ROI shape |

Additionally, candidate feature values may include values obtained through analysis of pixels or voxels. The values obtained through analysis include first order feature values obtained from the pixel histogram of the original image and second order feature values obtained from transformed information. Examples of the first order feature values are shown in [Table 3] below.

[Table 3]

|  | Name | Description |
|---|---|---|
| 1 | Energy | Measurement of the size of voxel value |
| 2 | Total Energy | Energy value scaled to voxel volume in cubic mm |
| 3 | Entropy | Measurement of average amount of information necessary to encode image value |
| 4 | Minimum | Minimum size of voxel value |
| 5 | 10th Percentile | Magnitude of voxel value corresponding 10-th percentile |
| 6 | 90th Percentile | Magnitude of voxel value corresponding 90-th percentile |
| 7 | Maximum | Maximum size of voxel values |
| 8 | Mean | Mean of voxel values |
| 9 | Median | Median value between maximum and minimum values of voxel values |
| 10 | Interquartile Range | Middle two ranges among quartered ranges between maximum and minimum values of voxel values |
| 11 | Range |  |
| 12 | Mean Absolute Deviation | Mean distance of all intensity values from mean of image array |
| 13 | Robust Mean Absolute Deviation | Mean distance of all intensity values from mean value calculated within 10-th to 90-th percentile |
| 14 | Root Mean Squared | Square root of the mean of all intensity squared values |
| 15 | Standard Deviation | Standard deviation of voxel values |
| 16 | Skewness | Measurement of asymmetry of value distribution of the mean value |
| 17 | Kurtosis | Measurement of sharpness of distribution |
| 18 | Variance | Variance of voxel values |
| 19 | Uniformity | Measurement of the sum of squares of each intensity value |

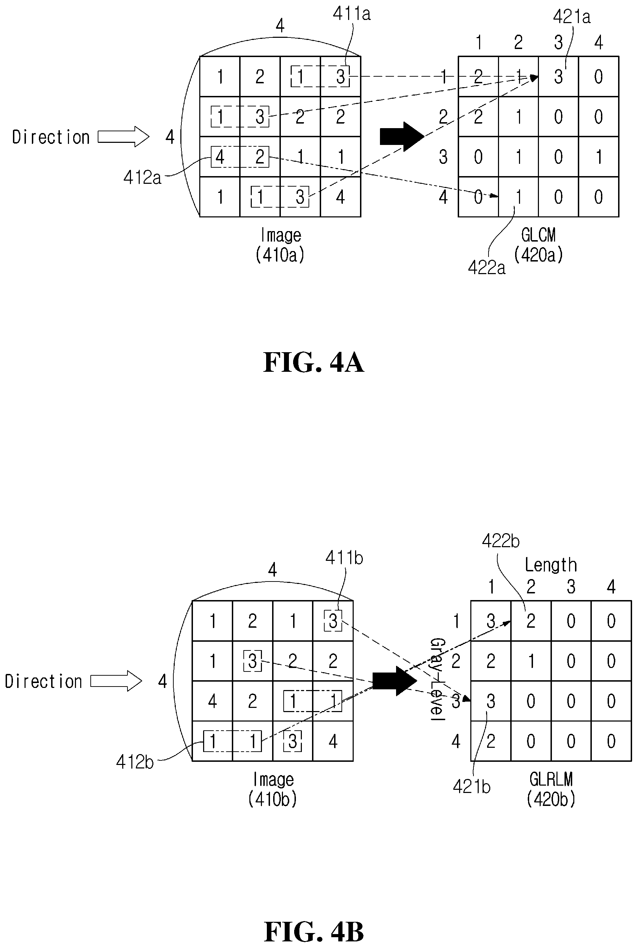

The second order feature values may be determined from information obtained by transforming an ultrasound image. Here, image transformation may be performed according to various techniques, and techniques that may express the texture of heart tissue from various perspectives may be used. By transforming the image, a feature matrix representing the textural characteristics of the image may be determined. For example, systems according to various embodiments may adopt feature matrices representing texture as shown in FIGS. 4A to 4E below.

FIGS. 4A to 4E show examples of feature matrices determined from an ultrasound image according to an embodiment of the present invention. The feature matrices illustrated in FIGS. 4A to 4E are for expressing the texture of muscle tissue of an ultrasound imaged heart, and are determined based on pixel values of the ultrasound image.

FIGS. 4A to 4E illustrate transformation for an image with a size of 4×4 or 5×5 for convenience of explanation, but the feature matrix may be determined in a similar manner for a ultrasound image with a larger size.

FIG. 4A illustrates a gray-level co-occurrence matrix (GLCM). Referring to FIG. 4A, in an image 410a, three patterns 411a where two adjacent pixel values are [1,3] are observed, and one pattern 412a where two adj acent pixel values are [4,2] is observed. In the case of FIG. 4A, two pixels are shown adj acent in the left and right directions, but being adj acent means being continuous in any one of the 0-degree direction, 45-degree direction, 90-degree direction, and 135-degree direction. Accordingly, adjacency may be evaluated in a different direction than the example in FIG. 4A. In the GLCM 420a, row numbers and column numbers correspond to combinations of pixel values that make up the pattern. Accordingly, an element 421a in the first row and third column of the GLCM 420a is set to 3, and an element 422a in the fourth row and second column is set to 1. The remaining elements are set according to the same rules. Examples of feature values that may be determined from the GLCM as shown in FIG. 4A are shown in Table 4 below.

[Table 4]

| | | Name | Description |
|---|---|---|---|
| | 1 | Autocorrelation | Measurement of texture precision and stenosis size |
| | 2 | Joint Average | Measurement of average Gray-Level Intensity of distribution |
| | 3 | Cluster Prominence | Measurement of skewness and asymmetry |
| | 4 | Cluster Shade | Measurement of skewness and uniformity |
| | 5 | Cluster Tendency | Measurement of grouping of voxels with similar Gray-Level values |
| | 6 | Contrast | Measurement of local Intensity fluctuations |
| | 7 | Correlation | Linear dependence value of gray-level value for each voxel |
| | 8 | Difference Average | Measurement of relationship between occurrence of pairs with similar intensity values and occurrence of pairs with different intensity values |
| | 9 | Difference Entropy | Measurement of randomness/variability from differences in adjacent intensity values |
| | 10 | Difference Variance | Measurement of heterogeneity by weighting different intensity values deviating more from the mean |
| | 11 | Joint Energy | Measurement homogeneous patterns in image |
| | 12 | Joint Entropy | Measurement of randomness/variability of adj acent intensity values |
| | 13 | Informational Measure of Correlation 1 | Quantification of texture complexity |
| | 14 | Informational Measure of Correlation 2 | Quantification of texture complexity |
| | 15 | Inverse Difference Moment | Measurement of local homogeneity of image |
| | 16 | Maximal Correlation Coefficient | Measurement of texture complexity |
| | 17 | Inverse Difference Moment Normalized | Measurement of local homogeneity of image |
| | 18 | Inverse Difference | Measurement of local homogeneity of image |
| | 19 | Inverse Difference Normalized Joint Energy | Measurement of local homogeneity of image and normalization of difference between adjacent intensity values |
| | 20 | Inverse Variance | |
| | 21 | Maximum Probability | Measurement of occurrence of the most dominant pair of adjacent intensity values |
| | 22 | Sum Average | Measurement of relationship between occurrence of pairs with low intensity value and occurrence of pairs with high intensity values |
| | 23 | Sum Entropy | Sum of differences between adj acent intensity values |

(continued)

|  | Name | Description |
|---|---|---|
| 24 | Sum of Squares | Measurement of distribution of adjacent intensity level pairs relative to average intensity level |

FIG. 4B illustrates a gray-level run length matrix (GLRLM). Referring to FIG. 4B, in an image 410b, three patterns 411b with a pixel value of 3 and a length of 1 are observed, and two patterns 412b with a pixel value of 1 and a length of 2 are observed. In the GLRLM 420b, row numbers correspond to pixel values (e.g., gray-level), and column numbers correspond to the length of the pattern. Accordingly, an element 421b in the third row and first column of the GLRLM 420b is set to 3, and an element 422b in the first row and second column is set to 2. The remaining elements are set according to the same rules. Examples of feature values that may be determined from the GLRLM as shown in FIG. 4B are shown in Table 5 below.

[Table 5]

|  | Name | Description |
|---|---|---|
| 1 | Short Run Emphasis | Measurement of distribution of short run length |
| 2 | Long Run Emphasis | Measurement of distribution of long run length |
| 3 | Gray Level Non-Uniformity | Measurement of similarity of gray-level intensity value of image |
| 4 | Gray Level Non-Uniformity Normalized | Normalization of similarity of gray-level intensity value of image |
| 5 | Run Length Non-Uniformity | Measurement of similarity of run length in entire image |
| 6 | Run Length Non-Uniformity Normalized | Normalization of similarity of run length in entire image |
| 7 | Run Percentage | Measurement of texture stenosis using ratio of the number of runs and the number of voxels |
| 8 | Gray Level Variance | Measurement of variance of gray-level intensity for run |
| 9 | Run Variance | Measurement of variance of run for run length |
| 10 | Run Entropy | Measurement of uncertainty /randomness in run length and gray-level intensity |
| 11 | Low Gray Level Run Emphasis | Measurement of distribution of low gray-level value |
| 12 | High Gray Level Run Emphasis | Measurement of distribution of high gray-level value |
| 13 | Short Run Low Gray Level Emphasis | Measurement of ratio in joint distribution images of short run lengths with low gray-level values |
| 14 | Short Run High Gray Level Emphasis | Measurement of ratio in joint distribution images of short run lengths with high gray-level values |
| 15 | Long Run Low Gray Level Emphasis | Measurement of ratio in joint distribution images of long run lengths with low gray-level values |
| 16 | Long Run High Gray Level Emphasis | Measurement of ratio in joint distribution images of long run lengths with high gray-level values |

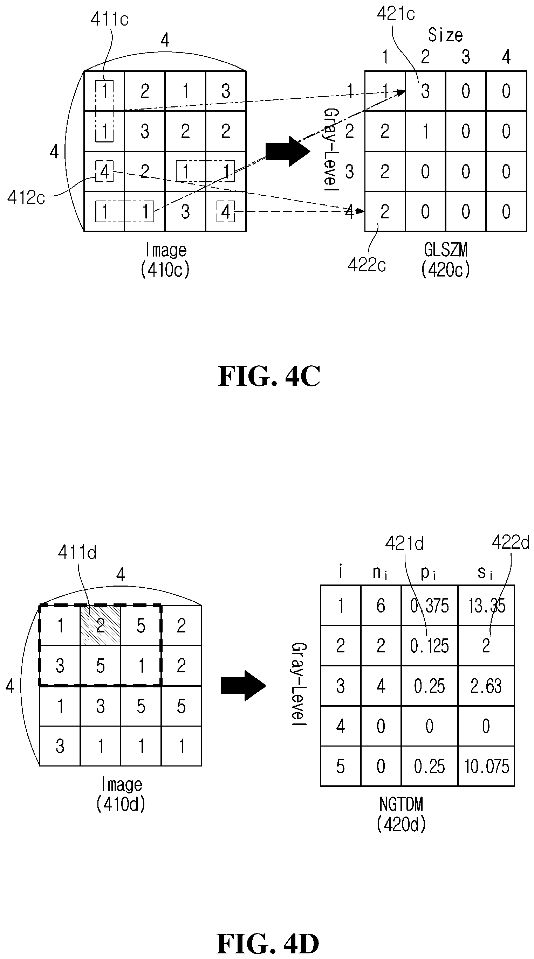

FIG. 4C illustrates a gray-level size zone matrix (GLSZM). Referring to FIG. 4C, in an image 410c, regardless of the direction, three patterns 411c with a pixel value of 1 and a length of 2 are observed, and two patterns 412c with a pixel value of 4 and a length of 1 are observed. In the GLSZM 420b, row numbers correspond to pixel values (e.g., gray-level), and column numbers correspond to the length of the pattern. Accordingly, an element 421c in the first row and second column of the GLSZM 420c is set to 3, and an element 422c in the fourth row and first column is set to 2. The remaining elements are set according to the same rules. Examples of feature values that may be determined from the GLSZM as shown in FIG. 4c are shown in Table 6 below.

[Table 6]

| | | Name | Description |
|---|---|---|---|
| | 1 | Small Area Emphasis | Measurement of distribution of small-sized area and measurement of line texture |
| | 2 | Large Area Emphasis | Measurement of distribution of large-sized area and measurement of rough texture |
| | 3 | Gray Level Non-Uniformity | Measurement of variability of gray-level intensity value of image |
| | 4 | Gray Level Non-Uniformity Normalized | Normalization of variability of gray-level intensity value of image |
| | 5 | Size-Zone Non-Uniformity | Measurement of variability of size zone volume within image |
| | 6 | Size-Zone Non-Uniformity Normalized | Normalization of variability measurement of volume in size zone within image |
| | 7 | Zone Percentage | Measurement of stenosis of texture using ratio of the number of zones and the number of voxels in ROI |
| | 8 | Gray Level Variance | Measurement of variance of gray-level intensity for zone |
| | 9 | Zone Variance | Measurement of variance of size zone volume |
| | 10 | Zone Entropy | Measurement of uncertainty /randomness in zone size and gray-level distribution |
| | 11 | Low Gray Level Zone Emphasis | Measurement of distribution of low gray-level size zone |
| | 12 | High Gray Level Zone Emphasis | Measurement of distribution of high gray-level size zone |
| | 13 | Small Area Low Gray Level Emphasis | Measurement of ratio in joint distribution images of small-sized area with low gray-level value |
| | 14 | Small Area High Gray Level Emphasis | Measurement of ratio in joint distribution images of small-sized area with high gray-level value |
| | 15 | Large Area Low Gray Level Emphasis | Measurement of ratio in joint distribution images of large-sized area with low gray-level value |
| | 16 | Large Area High Gray Level Emphasis | Measurement of ratio in joint distribution images of large-sized area with high gray-level value |

FIG. 4D illustrates a neighborhood gray-tone difference matrix (NGTDM). Referring to FIG. 4D, in the NGTDM 420d, row numbers correspond to pixel values (e.g., gray level), a first column represents a pixel value, a second column represents the number of pixels with the corresponding pixel value, and a third column represents a ratio of the corresponding pixel value among all pixels, and a fourth column represents a sum of the differences with neighboring pixel values within a distance $\delta$. In the example of FIG. 4D, the distance $\delta$ is 1. In the case of a pixel 411d in the first row and second column in an image 410d, the pixel value is 2, and the neighboring pixel values are 1, 5, 3, 5, and 1. And, in the image 410d, there are two pixels with a value of 2. Accordingly, in the NGTDM 420d, an element in the second row and second column is set to 2, and an element 421d in the second row and third column is set to 0.125 according to the operation shown in [Equation 1] below, and an element 422d in the second row and fourth column is set to 2 according to the operation shown in [Equation 2] below.

【Equation 1】

$$n_i/N = 2/16 = 0.125$$

In [Equation 1], $n_i$ denotes the number of pixels with a value of i, and N denotes the total number of pixels.

【Equation 2】

$$s_i = \sum_0^{n_i} |i - \overline{X_i}| = \left| 2 - \frac{(1+3+5+1+5)}{5} \right| + \left| 2 - \frac{(5+1+3)}{3} \right| = 2$$

In [Equation 2], $s_i$ denotes a sum of differences between neighboring pixel values with a value of i, i denotes a pixel value, $n_i$ denotes the number of pixels with a value of i, and $\overline{X_i}$ denotes an average of neighboring pixel values of the pixel with a value of i.

The remaining elements are set according to the same rules. Examples of feature values that may be determined from the NGTDM as shown in FIG. 4D are shown in [Table 7] below.

[Table 7]

|   | Name | Description |
|---|------|-------------|
| 1 | Coarseness | Measurement of average difference between central voxel and its neighbors and spatial rate of change |
| 2 | Contrast | Measurement of change in spatial intensity |
| 3 | Busyness | Measurement of change from one pixel to neighboring pixel |
| 4 | Complexity | Measurement of how many primitive components are in image |
| 5 | Strength | Measurement of primitive the higher it is, the better the primitive is visible |

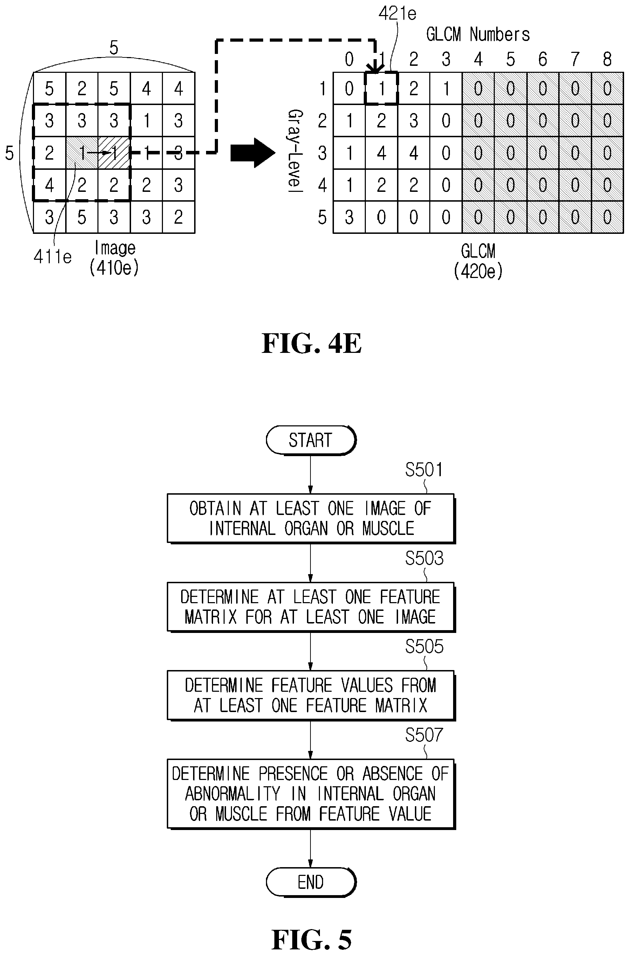

FIG. 4E illustrates a gray level dependence matrix (GLDM). Referring to FIG. 4E, in an image 410e, the value of the pixel 411e in the third row and second column is 1. Within the range where a distance $\delta$ is 1, there is one neighboring pixel with a difference a with the pixel 411e of 0. In the GLDM 420e, row numbers correspond to pixel values, and column numbers correspond to the distance $\delta$. Accordingly, an element 421e in the first row and second column of the GLDM 420e is set to 1. The remaining elements are set according to the same rules. Examples of feature values that may be determined from the GLDM as shown in FIG. 4E are shown in Table 8 below.

[Table 8]

|   | Name | Description |
|---|------|-------------|
| 1 | Small Dependence Emphasis | Measurement of distribution of small dependency |
| 2 | Large Dependence Emphasis | Measurement of distribution of large dependency |
| 3 | Gray Level Non-Uniformity | Measurement of similarity of gray level value in image |
| 4 | Dependence Non-Uniformity Normalized | Normalization of dependence similarity in entire image |
| 5 | Gray Level Variance | Measurement of variance of grey level |
| 6 | Dependence Variance | Measurement of variance in dependence size |
| 7 | Dependence Entropy | Measurement of entropy of dependence |
| 8 | Low Gray Level Emphasis | Measurement of distribution of low gray level value |
| 9 | High Gray Level Emphasis | Measurement of distribution of higher gray level value |
| 10 | Small Dependence High Gray Level Emphasis | Measurement of joint distribution of small dependence with higher gray level value |
| 11 | Large Dependence Low Gray Level Emphasis | Measurement of joint distribution of large dependence with lower gray level value |
| 12 | Large Dependence High Gray Level Emphasis | Measurement of joint distribution of large dependence with higher gray level value |
| 13 | Dependence Non-Uniformity | Measurement of dependence similarity in entire image |

(continued)

|  | Name | Description |
|---|---|---|
| 14 | Small Dependence Low Gray Level Emphasis | Measurement of joint distribution of small dependence with low gray level value |

In addition to the feature values determined from the above-described feature vectors, feature values obtained through a wavelet filter may be further considered. For example, after applying various wavelet filters to the obtained image, the above-described feature values may be generated for the filtered image.

According to various embodiments of the present invention, at least some of the above-described feature values may be used to determine presence or absence of abnormality in an internal organ or muscle. According to one embodiment, except for shape feature values and first order feature values, second order feature values and feature values based on the wavelet filter may be used.

FIG. 5 shows an example of a procedure for determining presence or absence of abnormality in an internal organ or muscle according to an embodiment of the present invention. FIG. 5 illustrates a method of operating a device with computing capabilities (e.g., the service server 110 of FIG. 1 or a separate server).

Referring to FIG. 5, in step S501, the device obtains at least one image of an internal organ or muscle. Here, the at least one image may be based on at least one of ultrasound, computed tomography (CT), positron emission tomography (PET), or magnetic resonance imaging (MRI). For example, the at least one image may be received from an external device through a wired or wireless communication connection. For example, when an object subjected to determination of presence or absence of abnormality is a heart, the at least one image may include an image captured in at least one view where the myocardium of the left ventricle may be observed (e.g., A2C, A4C, PLAX, PSAX- mid).

In step S503, the device determines at least one feature matrix for the at least one image. According to one embodiment, the device may determine the at least one feature matrix based on pixel values included in a region of interest extracted from the at least one image. For example, the region of interest may include at least one selected from a group consisting of the outer wall, medial wall, parietal peritoneum, fascia, intervisceral space, intracavitary, and intraperitoneal viscera, skeletal muscle, myocardium, and smooth muscle. For example, the region of interest may include at least one selected from a group consisting of the outer wall, medial wall, parietal peritoneum, fascia, skeletal muscle, myocardium, and smooth muscle of the internal organ, and at least one selected from a group consisting of intervisceral space, intracavitary, and intraperitoneal viscera. For example, the region of interest may include at least one selected from a group consisting of the outer wall, medial wall, parietal peritoneum, fascia, skeletal muscle, myocardium, and smooth muscle of the internal organ. Additionally, the feature matrices may include at least one of GLCM, GLRLM, GLSZM, NGTDM, or GLDM.

In step S505, the device determines feature values from the at least one feature matrix. In other words, the device may determine feature values for measuring a tissue phenotype from at least one feature matrix. For example, the determined feature values may include at least one of the features listed in [Table 4] to [Table 8] above.

In step S507, the device determines presence or absence of abnormality in an internal organ or muscle from the feature value. In other words, the device uses a trained artificial intelligence model to determine presence or absence of abnormality in the internal organ or muscle. Here, the feature values used to determine the presence or absence of abnormality include items selected as information representing the tissue phenotype of the internal organ or muscle, and selection of the items may be made by a machine learning model. That is, the items may be selected using the machine learning model among candidate feature values that may be determined from the at least one feature matrix.

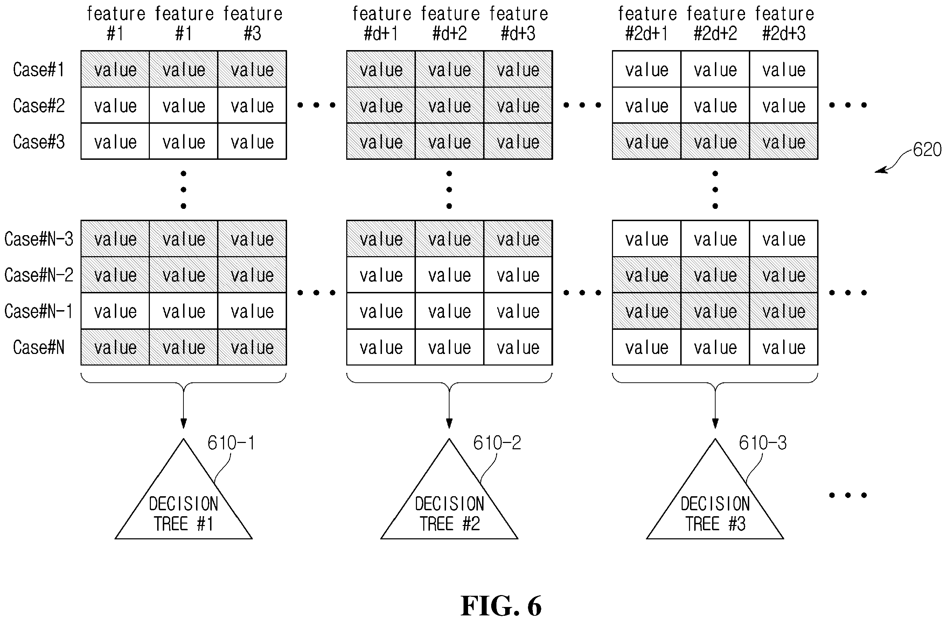

In the embodiment described with reference to FIG. 6, the items used to determine presence or absence of abnormality are selected using the machine learning model. For example, the machine learning model for selecting the feature items may include a random forest model. An example of the random forest model will be described in more detail with reference to FIG. 6 below.

FIG. 6 shows an example of a random forest model for selecting feature items according to an embodiment of the present invention. Referring to FIG. 6, the random forest model consists of a plurality of decision trees including decision tree #1 610-1, decision tree #2 610-2, and decision tree #3 610-3.

To train decision trees, the device generates a data pool 620 using feature values determined for each case. For example, the data pool 620 may include a matrix with as many rows as the number of cases and as many columns as the number of items of feature values. That is, each row of the data pool 620 corresponds to each case, and each column corresponds to each feature item. Thereafter, the device extracts subsets of data from the data pool 620 to generate a learning data set for each of the decision trees.

At this time, according to one embodiment, duplication of cases may be allowed, but duplication of feature values may not be allowed. In this case, for each decision tree, the device generates input data sets by extracting n cases under duplication conditions and extracting d feature values for the extracted cases without duplication. For example, as shown in FIG. 6, learning data for decision tree #1 610-1 may include feature values #1 to #d of case #1, case #N-3, case #N-2, and case #N.

The device may calculate the Gini index for each node of the generated decision trees. In other words, the device may determine the impurity for each node of the generated decision trees. In addition, based on the calculated Gini index, K feature items with relatively high importance may be selected. The selected K feature items are used as items to select feature values used for later diagnosis.

According to one embodiment, the device may sort the feature values in order of importance based on the Gini index, identify a point where an area under the curve (AUC) is saturated while the feature values with the highest importance is added one by one to the random forest model trained for diagnosis, and determine the number of feature value items to be used for diagnosis based on the identified point. For example, when checking the AUC of the model by applying 1 to 10 feature values, the AUC continues to increase, but if the increase in AUC as more feature values are added from 11 is not significant, the device may finally select ten feature value items.

As described above, the tissue phenotype of an organ or muscle may be measured based on feature values extracted from the image, and the presence or absence of abnormality in the organ or muscle may be determined based on the measured phenotype. According to one embodiment, the above-described procedure of determining presence or absence of the abnormality may be performed on the heart using ultrasound images. In other words, the above-described technology of determining presence or absence of the abnormality based on the phenotype may be performed to determine the presence or absence of heart disease using ultrasound images. Hereinafter, an embodiment of diagnosing heart disease based on phenotypic measurement will be described. However, it is obvious that the embodiments described below may be performed for the diagnosis of diseases other than heart disease.

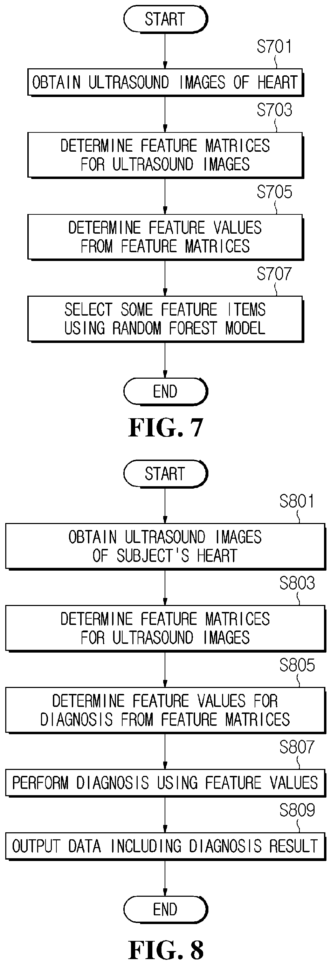

FIG. 7 shows an example of a procedure for selecting feature items for diagnosis of heart disease according to an embodiment of the present invention. FIG. 7 illustrates a method of operating a device with computing capabilities (e.g., the service server 110 of FIG. 1 or a separate server).

Referring to FIG. 7, in step S701, the device obtains ultrasound images of the heart. The ultrasound images may be received from an external device through a wired or wireless communication connection. For example, the view of ultrasound images may include at least one of A2C, A4C, PLAX, or PSAX-mid. According to one embodiment, the obtained ultrasound images may include images of cases with heart disease and images of cases without heart disease at a ratio of 1:1.

In step S703, the device determines feature matrices for the ultrasound images. According to one embodiment, the feature matrices may be determined for some of the ultrasound images. Specifically, the device may extract a region of interest from the ultrasound images and determine feature matrices by transforming pixel values within the extracted region of interest. For example, the region of interest may include at least one of the left ventricular (LV) wall or the left ventricular cavity. Additionally, the feature matrices may include at least one of GLCM, GLRLM, GLSZM, NGTDM or GLDM.

In step S705, the device determines feature values from the feature matrices. For example, the determined feature values may include at least one of the feature values listed in [Table 4] to [Table 8] above.

In step S707, the device selects some feature items using a random forest model. The random forest model for selecting feature items includes a plurality of decision trees, and each of the decision trees classifies disease status by using some of the feature values determined in step S705 without mutual duplication. The device may determine the Gini index for the classification results from each of the decision trees, and identify K feature items with the lowest impurity based on the Gini index. The random forest model for selecting feature items will be explained in more detail with reference to 8 below.

FIG. 8 shows an example of a procedure for diagnosing heart disease using an artificial intelligence model according to an embodiment of the present invention. FIG. 8 illustrates a method of operating a device with computing capabilities (e.g., the service server 110 of FIG. 1).

Referring to FIG. 8, in step S801, the device obtains ultrasound images of a subject's heart. The ultrasound images may be received from an external device through a wired or wireless communication connection. For example, the view of the ultrasound images may include at least one of A2C, A4C, PLAX or PSAX-mid. According to one embodiment, the ultrasound images may be provided in real time from ultrasound imaging equipment.

In step S803, the device determines feature matrices for the ultrasound images. According to one embodiment, the feature matrices may be determined for some of the ultrasound images. Specifically, the device may extract a region of interest from the ultrasound images and determine feature matrices by transforming the extracted region of interest. For example, the region of interest may include at least one of the left ventricular wall or the left ventricular cavity. Additionally, the feature matrices may include at least one of GLCM, GLRLM, GLSZM, NGTDM, or GLDM.

In step S805, the device determines feature values for diagnosis from the feature matrices. According to one embodiment, the feature values determined in this step may be limited to items previously selected for diagnosis. The selected items include feature values that were determined to represent the phenotype enough to diagnose heart disease. For example, the selected items may include feature values selected using a random forest model.

In step S807, the device performs diagnosis using the determined feature values. For example, the device may predict whether heart disease occurs using a trained artificial intelligence model, such as the random forest model trained for diagnosis. The random forest model for diagnosis includes decision trees trained using feature values of selected items. To this end, the device may input the feature values determined from the ultrasound images for each view to the random forest model, check the classification results of decision trees included in the random forest model, and combine the checked classification results to determine a final diagnosis result.

In step S809, the device outputs data including the diagnosis result. According to one embodiment, the device may generate data indicating the diagnosis result for heart disease and transmit the generated data to a client device. Accordingly, the client device may receive data, check the diagnosis result of heart disease based on the received data, visualize it (e.g., display, print, etc.), or send it to the subject (e.g., email, upload, etc.).

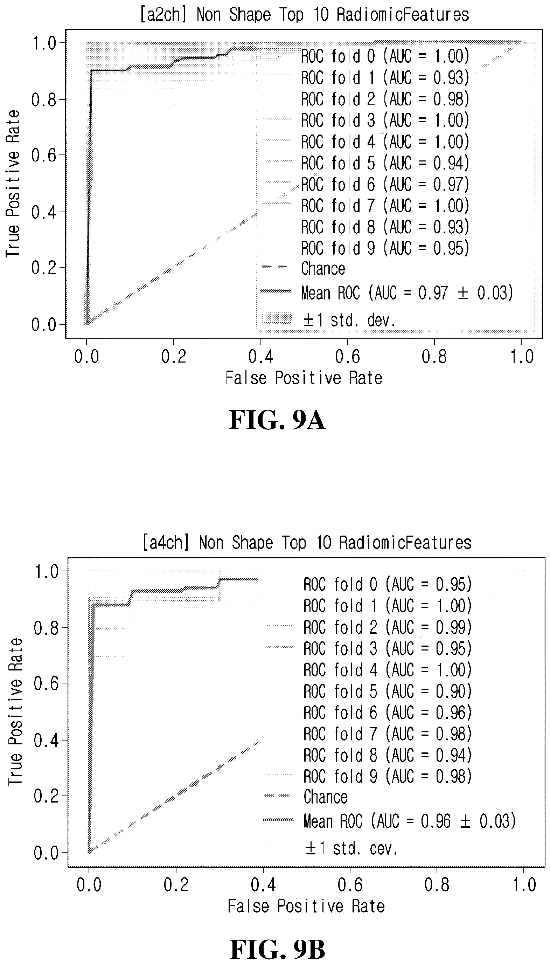

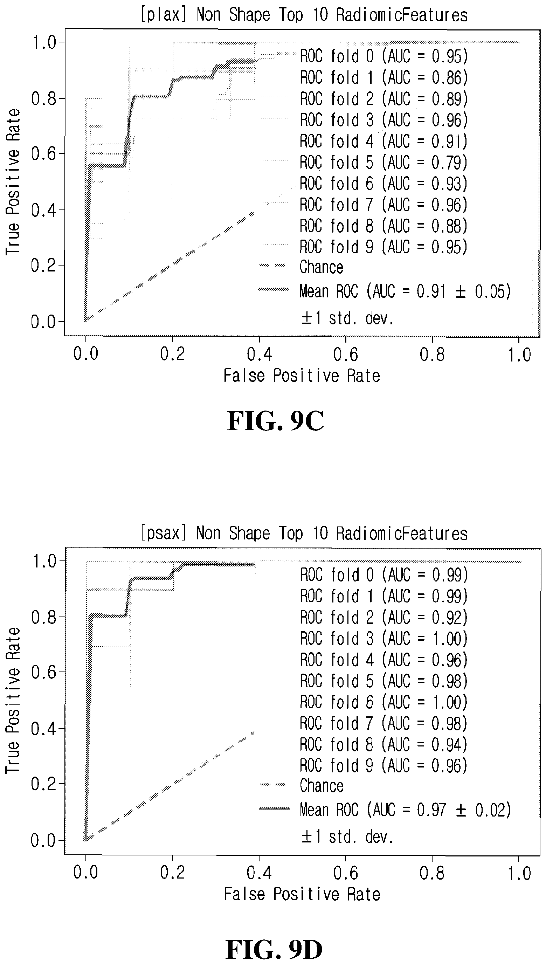

Heart disease may be diagnosed based on the texture of the heart tissue as in the various embodiments described above. The results of verifying the artificial intelligence model used to diagnose heart disease using 10-fold cross validation and leave-one-out (LOO) cross validation methods will be described with reference to FIGS. 9A to 15 below.

FIGS. 9A to 9D show validation results for an artificial intelligence model according to a 10-fold cross validation method when using an image of an end systole phase according to an embodiment of the present invention. FIGS. 9A to 9D show receiver operating characteristic (ROC) curves for each view of A2CH, A4CH, PLAX, and PSAX. As shown in FIGS. 9A to 9D, an average area under the curve (AUC) exceeding 0.9 is confirmed in all views.

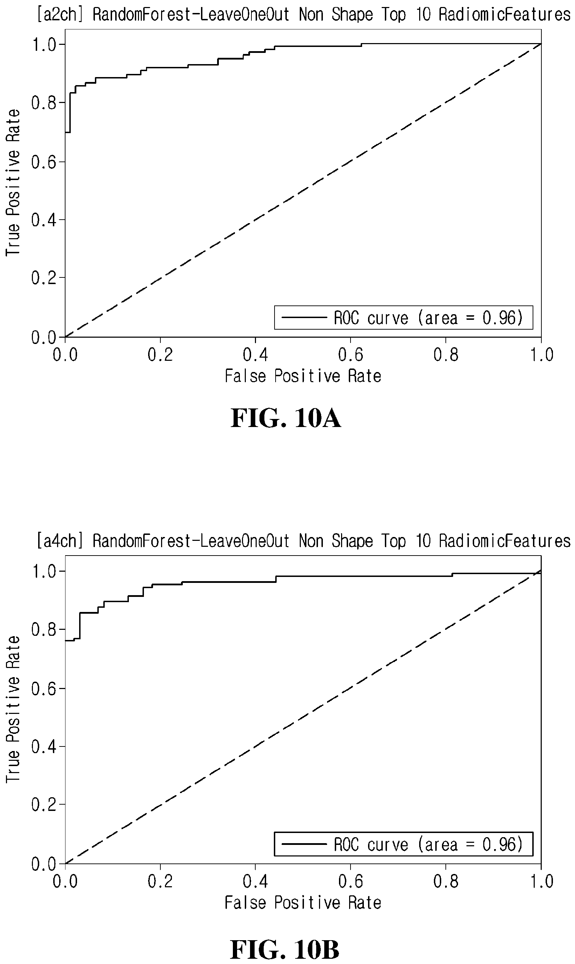

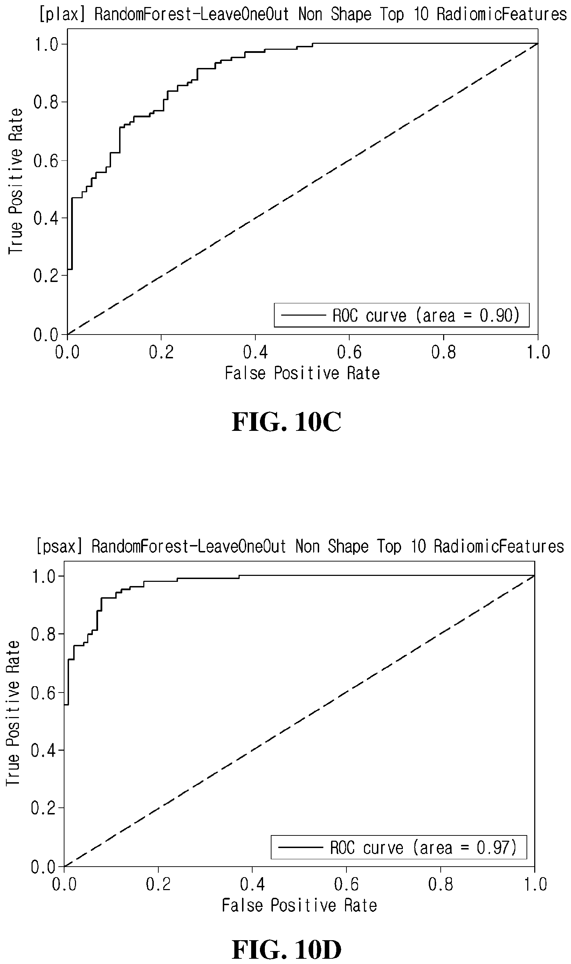

FIGS. 10A to 10D show verification results for an artificial intelligence model according to an LOO cross validation method when using an image of the end systole phase according to an embodiment of the present invention. FIGS. 10A to 10D show ROC curves for each view of A2CH, A4CH, PLAX, and PSAX. As shown in FIGS. 10A to 10D, an AUC of 0.9 or more is confirmed in all views.

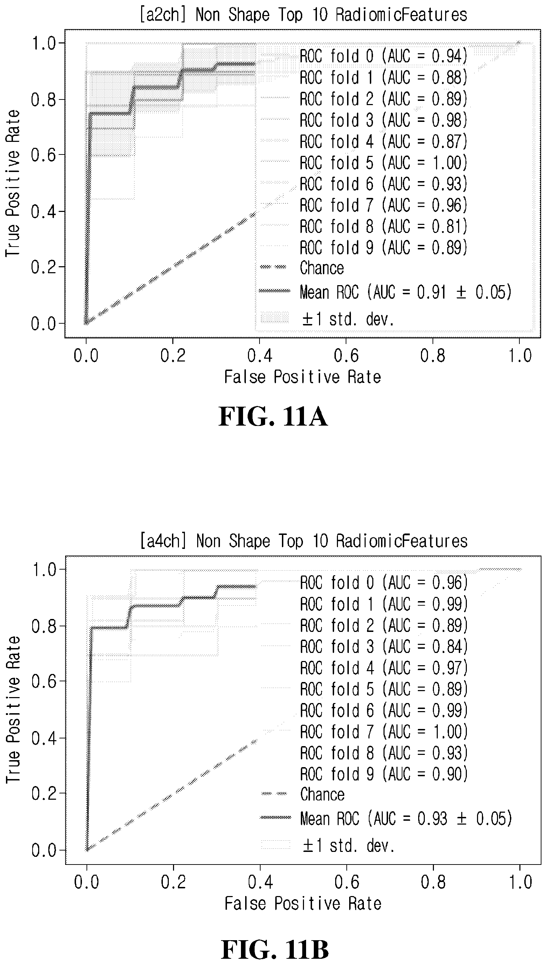

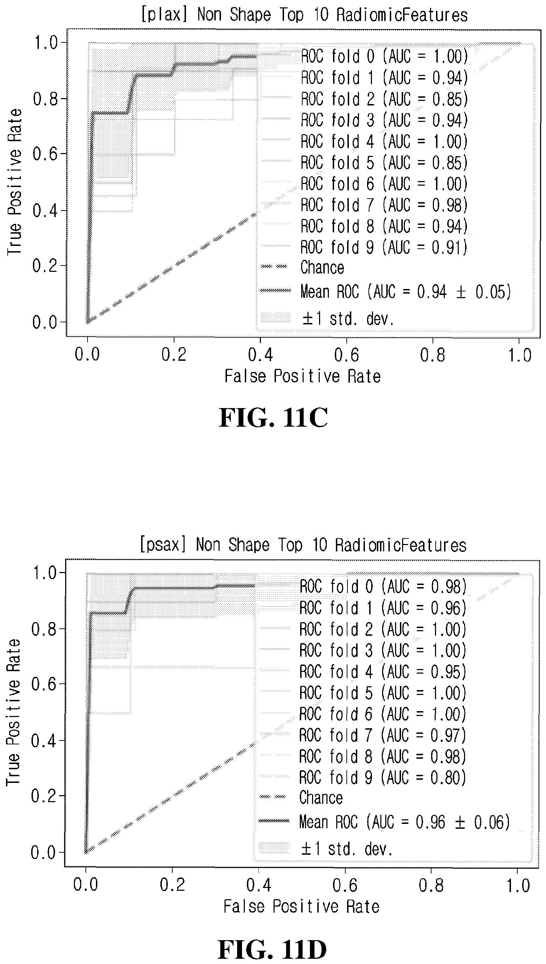

FIGS. 11A to 11D show verification results for an artificial intelligence model according to a 10-fold cross validation method when using an image of an end diastole phase according to an embodiment of the present invention. FIGS. 11A to 11D show ROC curves for each view of A2CH, A4CH, PLAX, and PSAX. As shown in FIGS. 11A to 11D, an average AUC exceeding 0.9 is confirmed in all views.

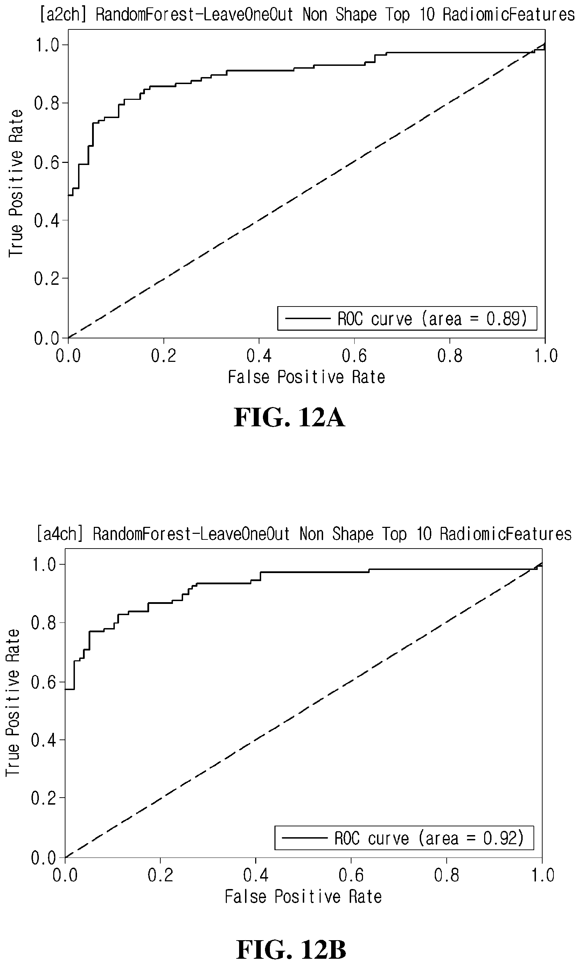

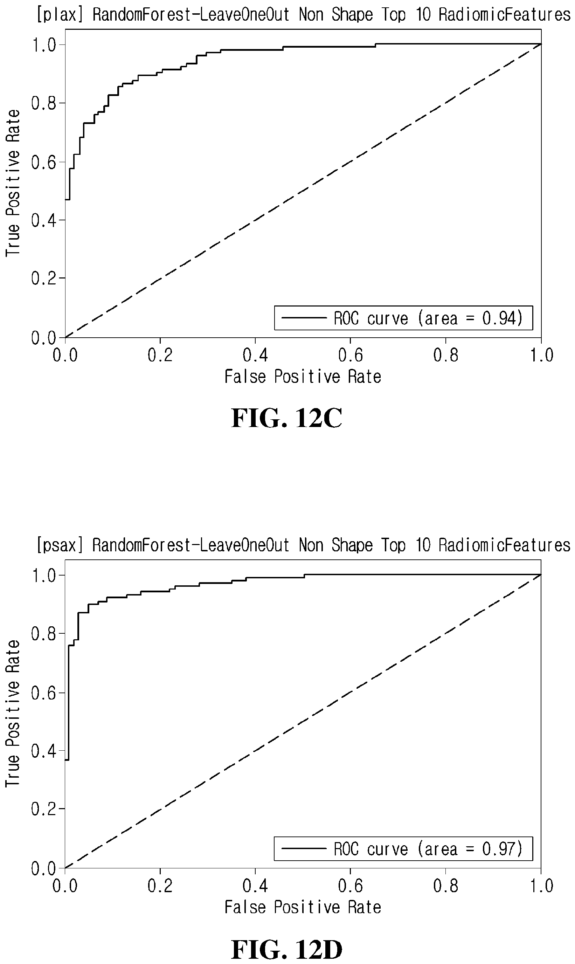

FIGS. 12A to 12D show verification results for an artificial intelligence model according to an LOO cross validation method when using an image of an end diastole phase according to an embodiment of the present invention. FIGS. 12A to 12D show ROC curves for each view of A2CH, A4CH, PLAX, and PSAX. As shown in FIGS. 12A to 12D, an AUC of approximately 0.9 is confirmed in all views.

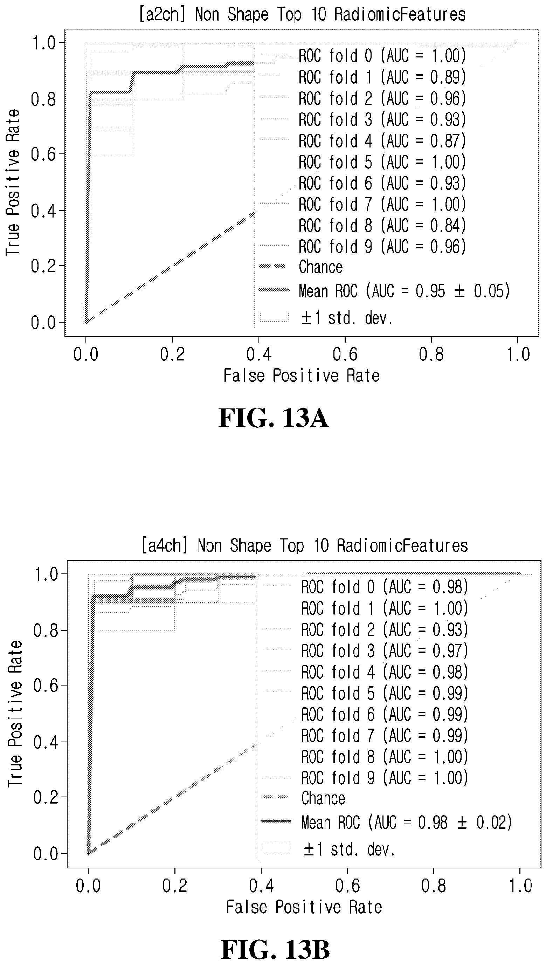

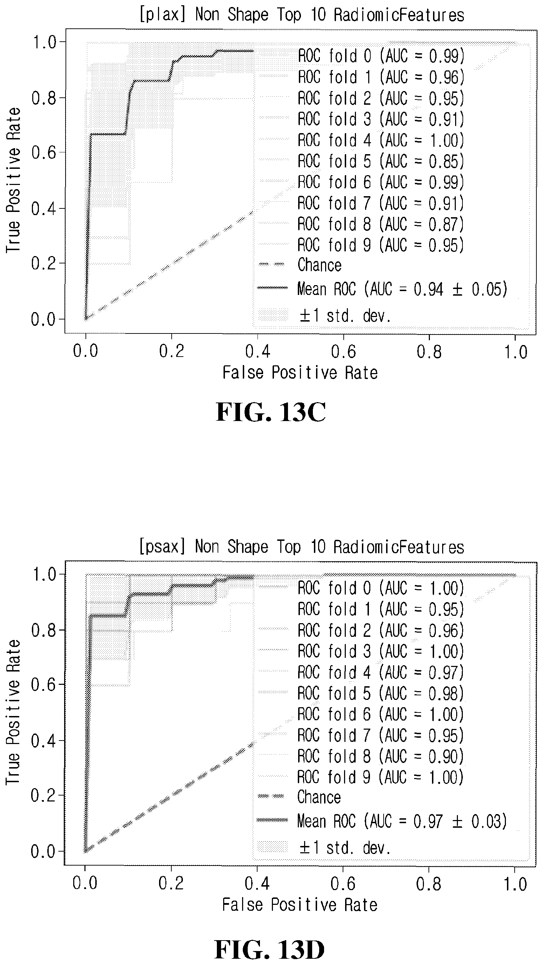

FIGS. 13A to 13D show verification results for an artificial intelligence model according to a 10-fold cross validation method when using a mid-phase image according to an embodiment of the present invention. FIGS. 13A to 13D show ROC curves for each view of A2CH, A4CH, PLAX, and PSAX. As shown in FIGS. 13A to 13D, an average AUC exceeding 0.9 is confirmed in all views.

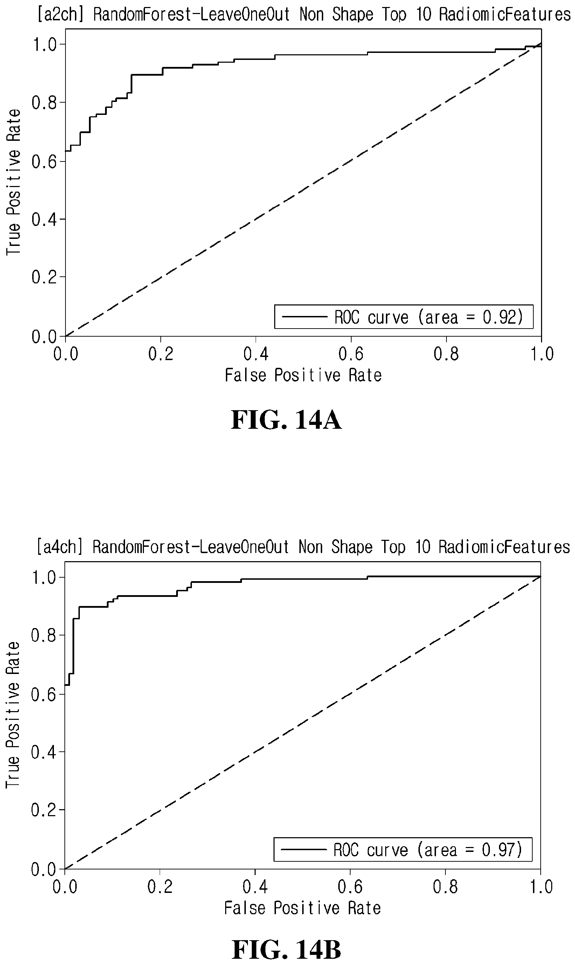

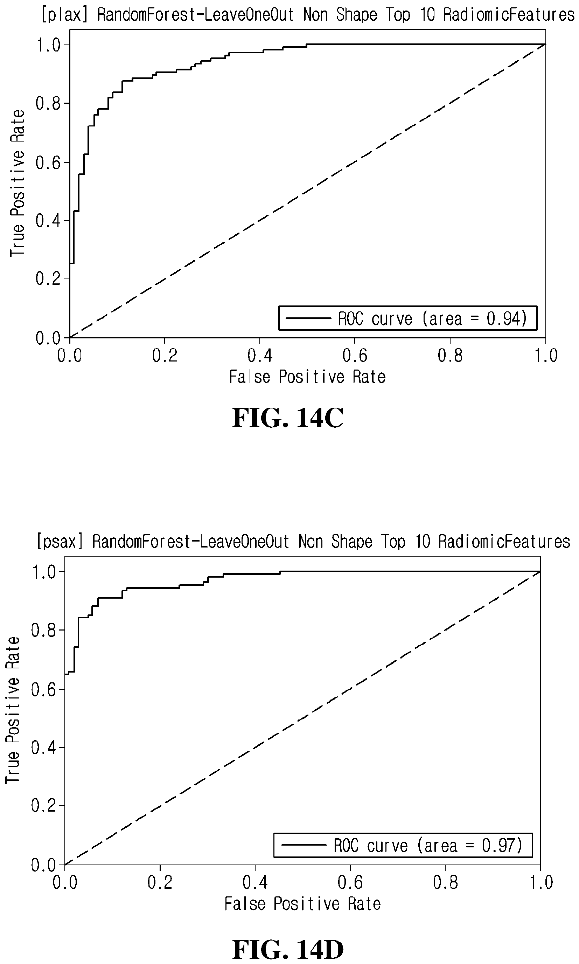

FIGS. 14A to 14D show verification results for an artificial intelligence model according to an LOO cross validation method when using an image of a mid-phase according to an embodiment of the present invention. FIGS. 14A to 14D show ROC curves for each view of A2CH, A4CH, PLAX, and PSAX. As shown in FIGS. 14A to 14D, an average AUC exceeding 0.9 is confirmed in all views.

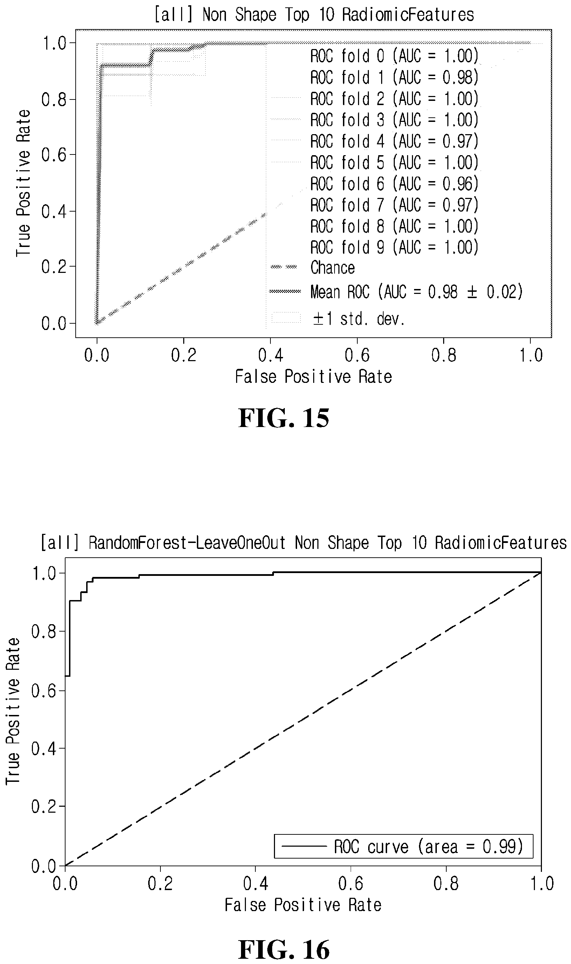

FIG. 15 shows verification results for an artificial intelligence model according to an 10-fold cross validation method when using an image of all phases according to an embodiment of the present invention. As shown in FIG. 15, an average AUC of 0.98 is confirmed.

FIG. 16 shows verification results for an artificial intelligence model according to an LOO cross validation method when using an image of all phases according to an embodiment of the present invention. As shown in FIG. 15, an AUC of 0.99 is confirmed.

While the exemplary methods of the present disclosure described above are represented as a series of operations for clarity of description, it is not intended to limit the order in which the steps are performed, and the steps may be performed simultaneously or in different order as necessary. In order to implement the method according to the present disclosure, the described steps may further include other steps, may include remaining steps except for some of the steps, or may include other additional steps except for some steps.

The various embodiments of the present disclosure are not a list of all possible combinations and are intended to describe representative aspects of the present disclosure, and the matters described in the various embodiments may be applied independently or in combination of two or more.

Various embodiments of the present disclosure may be implemented in hardware, firmware, software, or a combination thereof. In the case of implementing the present disclosure by hardware, the present disclosure can be implemented with application specific integrated circuits (ASICs), Digital signal processors (DSPs), digital signal processing devices (DSPDs), programmable logic devices (PLDs), field programmable gate arrays (FPGAs), general processors, controllers, microcontrollers, microprocessors, etc.

The scope of the present disclosure includes a non-transitory computer-readable medium in which software or machine-executable instructions (e.g., operating system, application, firmware, program, etc.) that cause operations according to the methods of various embodiments to be executed on a device or computer and such software or instructions are stored and may be executed on a device or computer.

**Claims**

1. A method of determining presence or absence of abnormality in an internal organ or muscle, the method comprising:

   obtaining at least one image of the internal organ or muscle;
   determining at least one feature matrix for the at least one image;
   determining a feature value for measuring a tissue phenotype from the at least one feature matrix; and
   determining presence or absence of the abnormality in the internal organ or muscle using a trained artificial intelligence model,
   wherein the feature value comprises items selected as information indicating the tissue phenotype of the internal organ or muscle, and
   wherein the items are selected using a machine learning model among candidate feature values determinable from the at least one feature matrix.

2. The method of claim 1, wherein the at least one image comprises at least one of ultrasound, computed tomography (CT), positron emission tomography (PET), or magnetic resonance imaging (MRI).

3. The method of claim 1, wherein the at least one image comprises an image captured in at least one view of apical 2-chamber (A2CH), apical 4-chamber (A4CH), parasternal long-axis (PLAX), parasternal short-axis (PSAX)-mid, PLAX increased depth, parasternal long axis left ventricle (PLAX LV), parasternal long axis left ventricle aorta/left atrial (PLAX LV-AO/LA), parasternal long axis zoomed left ventricle (PLAX zoomed LV), parasternal long axis zoomed aortic valve (PLAX zoomed AV), parasternal long axis zoomed mitral valve (PLAX zoomed MV), parasternal long axis right ventricle (PLAX RV) out flow, parasternal long axis right ventricle (PLAX RV) inflow, parasternal short axis (level great vessels) focus on pulmonic valve (PSAX focus on PV), parasternal short axis (level great vessels) focus on aortic valve (PSAX focus on AV), parasternal short axis (level great vessels) zoomed aortic valve(PSAX zoomed AV), parasternal short axis (level great vessels) focus on tricuspid valve (PSAX (level great vessels) focus on TV), parasternal short axis focus on pulmonic valve and pulmonary artery (PSAX focus on PV and PA), parasternal short axis-level of mitral valve (PSAX-level of MV), parasternal short axis(PSAX)-level of papillary muscles, parasternal short axis (PSAX)-level of apex, A5C (apical five-chamber), A4C (apical four-chamber), apical four-chamber zoomed left ventricle (A4C zoomed LV), apical four-chamber right ventricle(A4C RV-focused)-focused, apical four-chamber (A4C) posterior angulation), apical three-chamber (A3C), apical three-chamber zoomed left ventricle (A3C zoomed LV), apical two-chamber (A2C), apical two-chamber zoomed left ventricle (A2C zoomed LV), apical long axis, apical long axis zoomed left ventricle (LV), apical four-chamber left atrial pulmonary vein focus (A4C LA Pulvn), subcostal four-chamber (SC 4C), subcostal long axis inferior vena cava (SC long axis IVC), subcostal window hepatic vein (SC window Hvn), or suprasternal notch (SSN) aortic arch.

4. The method of claim 1, wherein the at least one feature matrix is determined based on pixel values included in a region of interest extracted from the at least one image.

5. The method of claim 4, wherein the region of interest comprises at least one selected from a group consisting of an outer wall, medial wall, parietal peritoneum, fascia, intervisceral space, intracavitary, and intraperitoneal viscera, skeletal muscle, myocardium, and smooth muscle.

6. The method of claim 1, wherein the at least one feature matrix comprises at least one of gray-level co-occurrence matrix (GLCM), gray-level run length matrix (GLRLM), gray-level size zone matrix (GLSZM), neighborhood gray-tone difference matrix (NGTDM) or gray level dependence matrix (GLDM).

7. The method of claim 1, wherein the abnormality in the internal organ or muscle comprises heart disease.

8. A device for determining presence or absence of abnormality in an internal organ or muscle, the device comprising:

   a transceiver;
   a storage unit configured to store an artificial intelligence model; and

at least one processor connected to the transceiver and the storage unit,
wherein the at least one processor is configured to:

obtain at least one image of the internal organ or muscle;
determine at least one feature matrix for the at least one image;
determine a feature value for measuring a tissue phenotype from the feature matrix; and
determine presence or absence of the abnormality in the internal organ or muscle using a trained artificial intelligence model,
wherein the feature values comprises items selected as information indicating the tissue phenotype in the internal organ or muscle, and
wherein the items are selected using a machine learning model among candidate feature values determinable from the at least one feature matrix.

**9.** A program stored on a medium to execute the method according to any one of claims 1 to 11 when operated by a processor.

**FIG. 1**

210

230

220

communication
unit

controller

storage unit

**FIG. 2**

**FIG. 3**

**FIG. 4A**

**FIG. 4B**

FIG. 4C

FIG. 4D

**FIG. 4E**

START

OBTAIN AT LEAST ONE IMAGE OF
INTERNAL ORGAN OR MUSCLE — S501

DETERMINE AT LEAST ONE FEATURE
MATRIX FOR AT LEAST ONE IMAGE — S503

DETERMINE FEATURE VALUES FROM
AT LEAST ONE FEATURE MATRIX — S505

DETERMINE PRESENCE OR ABSENCE OF
ABNORMALITY IN INTERNAL ORGAN
OR MUSCLE FROM FEATURE VALUE — S507

END

**FIG. 5**

|  | feature #1 | feature #1 | feature #3 |  | feature #d+1 | feature #d+2 | feature #d+3 |  | feature #2d+1 | feature #2d+2 | feature #2d+3 |  |
|---|---|---|---|---|---|---|---|---|---|---|---|---|
| Case#1 | value | value | value | • • • | value | value | value | • • • | value | value | value | • • • |
| Case#2 | value | value | value |  | value | value | value |  | value | value | value | • • • |
| Case#3 | value | value | value |  | value | value | value |  | value | value | value |  |
| Case#N-3 | value | value | value |  | value | value | value |  | value | value | value |  |
| Case#N-2 | value | value | value | • • • | value | value | value | • • • | value | value | value | • • • |
| Case#N-1 | value | value | value |  | value | value | value |  | value | value | value |  |
| Case#N | value | value | value |  | value | value | value |  | value | value | value |  |

620

610-1

610-2

610-3

DECISION TREE #1

DECISION TREE #2

DECISION TREE #3

• • •

**FIG. 6**

EP 4 404 206 A1

START

S701
OBTAIN ULTRASOUND IMAGES OF HEART

S703
DETERMINE FEATURE MATRICES
FOR ULTRASOUND IMAGES

S705
DETERMINE FEATURE VALUES
FROM FEATURE MATRICES

S707
SELECT SOME FEATURE ITEMS
USING RANDOM FOREST MODEL

END

**FIG. 7**

START

S801
OBTAIN ULTRASOUND IMAGES
OF SUBJECT'S HEART

S803
DETERMINE FEATURE MATRICES
FOR ULTRASOUND IMAGES

S805
DETERMINE FEATURE VALUES FOR
DIAGNOSIS FROM FEATURE MATRICES

S807
PERFORM DIAGNOSIS USING FEATURE VALUES

S809
OUTPUT DATA INCLUDING DIAGNOSIS RESULT

END

**FIG. 8**

FIG. 9A

FIG. 9B

[plax] Non Shape Top 10 RadiomicFeatures

**FIG. 9C**

[psax] Non Shape Top 10 RadiomicFeatures

**FIG. 9D**

[a2ch] RandomForest-LeaveOneOut Non Shape Top 10 RadiomicFeatures

**FIG. 10A**

[a4ch] RandomForest-LeaveOneOut Non Shape Top 10 RadiomicFeatures

**FIG. 10B**

[plax] RandomForest-LeaveOneOut Non Shape Top 10 RadiomicFeatures

**FIG. 10C**

[psax] RandomForest-LeaveOneOut Non Shape Top 10 RadiomicFeatures

**FIG. 10D**

[a2ch] Non Shape Top 10 RadiomicFeatures

**FIG. 11A**

[a4ch] Non Shape Top 10 RadiomicFeatures

**FIG. 11B**

[plax] Non Shape Top 10 RadiomicFeatures

**FIG. 11C**

[psax] Non Shape Top 10 RadiomicFeatures

**FIG. 11D**

[a2ch] RandomForest-LeaveOneOut Non Shape Top 10 RadiomicFeatures

ROC curve (area = 0.89)

**FIG. 12A**

[a4ch] RandomForest-LeaveOneOut Non Shape Top 10 RadiomicFeatures

ROC curve (area = 0.92)

**FIG. 12B**

**FIG. 12C**

**FIG. 12D**

[a2ch] Non Shape Top 10 RadiomicFeatures

FIG. 13A

[a4ch] Non Shape Top 10 RadiomicFeatures

FIG. 13B

[plax] Non Shape Top 10 RadiomicFeatures

**FIG. 13C**

[psax] Non Shape Top 10 RadiomicFeatures

**FIG. 13D**

[a2ch] RandomForest-LeaveOneOut Non Shape Top 10 RadiomicFeatures

FIG. 14A

[a4ch] RandomForest-LeaveOneOut Non Shape Top 10 RadiomicFeatures

FIG. 14B

[plax] RandomForest-LeaveOneOut Non Shape Top 10 RadiomicFeatures

FIG. 14C

[psax] RandomForest-LeaveOneOut Non Shape Top 10 RadiomicFeatures

FIG. 14D

**FIG. 15**

**FIG. 16**

## INTERNATIONAL SEARCH REPORT

| International application No. |
|---|
| **PCT/KR2022/013534** |

### A. CLASSIFICATION OF SUBJECT MATTER

**G16H 30/40**(2018.01)i; **G16H 30/20**(2018.01)i; **G16H 50/50**(2018.01)i; **G16H 50/20**(2018.01)i; **A61B 8/08**(2006.01)i; **A61B 6/00**(2006.01)i; **A61B 5/055**(2006.01)i

According to International Patent Classification (IPC) or to both national classification and IPC

### B. FIELDS SEARCHED

Minimum documentation searched (classification system followed by classification symbols)

G16H 30/40(2018.01); A61B 5/00(2006.01); G06F 19/00(2011.01); G06K 9/62(2006.01); G06T 7/00(2006.01); G16H 50/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인공지능(artificial intelligence), 알고리즘(algorithm), 의료영상(medical imaging), 조직(tissue), 분류(classification), 진단(diagnosis)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0040287 A (HEALTHHUB CO., LTD.) 20 April 2018 (2018-04-20)<br>See paragraphs [0056]-[0073]; claims 1, 12-14, 17 and 21-22; and figures 2-5 and 9. | 1-2,4,6-7,9 |
| Y | | 3,5,8 |
| Y | KR 10-2237198 B1 (DEEPNOID CO., LTD.) 08 April 2021 (2021-04-08)<br>See paragraphs [0017] and [0032]-[0040]; and figure 2. | 3,5,8 |
| A | KR 10-2017-0017614 A (WONKWANG UNIVERSITY CENTER FOR INDUSTRY-ACADEMY COOPERATION) 15 February 2017 (2017-02-15)<br>See paragraphs [0024]-[0069]; claims 1-7; and figures 1-4. | 1-9 |
| A | KR 10-2132375 B1 (KOREA ADVANCED INSTITUTE OF SCIENCE AND TECHNOLOGY et al.) 09 July 2020 (2020-07-09)<br>See paragraphs [0019]-[0184]; claims 1-2, 4-12 and 14-20; and figures 1-9. | 1-9 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 December 2022** | **08 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2022/013534** |

**C.    DOCUMENTS CONSIDERED TO BE RELEVANT**

| Category* | Citation of document, with indication, where appropriate, of the relevant passages | Relevant to claim No. |
|---|---|---|
| A | US 2021-0056693 A1 (TENCENT TECHNOLOGY (SHENZHEN) COMPANY LIMITED) 25 February 2021 (2021-02-25)<br>See paragraphs [0081]-[0216]; claims 1-20; and figures 1-9. | 1-9 |

Form PCT/ISA/210 (second sheet) (July 2022)

**INTERNATIONAL SEARCH REPORT**
Information on patent family members

International application No.

**PCT/KR2022/013534**

| Patent document cited in search report | | | Publication date (day/month/year) | Patent family member(s) | | | Publication date (day/month/year) |
|---|---|---|---|---|---|---|---|
| KR | 10-2018-0040287 | A | 20 April 2018 | KR | 10-1880678 | B1 | 20 July 2018 |
| KR | 10-2237198 | B1 | 08 April 2021 | | None | | |
| KR | 10-2017-0017614 | A | 15 February 2017 | KR | 10-1716039 | B1 | 13 March 2017 |
| KR | 10-2132375 | B1 | 09 July 2020 | CN | 114127858 | A | 01 March 2022 |
| | | | | EP | 3996103 | A1 | 11 May 2022 |
| | | | | WO | 2021-006522 | A1 | 14 January 2021 |
| US | 2021-0056693 | A1 | 25 February 2021 | CN | 109523526 | A | 26 March 2019 |
| | | | | CN | 109523526 | B | 22 October 2021 |
| | | | | EP | 3879485 | A1 | 15 September 2021 |
| | | | | JP | 2021-520568 | A | 19 August 2021 |
| | | | | JP | 7086336 | B2 | 20 June 2022 |
| | | | | WO | 2020-094026 | A1 | 14 May 2020 |

Form PCT/ISA/210 (patent family annex) (July 2022)